# EUROPÄISCHE PATENTSCHRIFT

(11) **EP 1 072 574 B1**
(45) Veröffentlichungstag und Bekanntmachung des Hinweises auf die Patenterteilung: **10.03.2004**
(21) Anmeldenummer: 00112842.0
(22) Anmeldetag: 17.06.2000
(51) Int. Cl.: C05F 17/02, C05F 9/04, C12M 1/107, C12M 1/00, C02F 11/04

(54) **Vergärungsanlage mit Vorrichtung zur Entwässerung von Fermentationsprodukten**
Fermentation plant with device for the dehydration of fermentation products
Installation de fermentation avec un dispositif de deshydratation des produits de fermentation

(30) Priorität: 26.07.1999 CH 136699
(43) Veröffentlichungstag der Anmeldung: 31.01.2001
(73) Patentinhaber: Walter Schmid AG, 8152 Glattbrugg (CH)
(72) Erfinder: Kern, Daniel, 8302 Kloten (CH)
(74) Vertreter: Feldmann, Clarence Paul

(56) Entgegenhaltungen:
- EP-A- 1 114 798
- DE-A- 4 427 644
- US-A- 5 782 950

## Beschreibung

Die vorliegende Erfindung betrifft eine Vergärungsanlage mit einem Fermenter und einer Entwässerungsvorrichtung für Fermentationsprodukte gemass Oberbegriff des Patentanspruches 1 und ein Entwässerungsverfahren für solche Fermentationsprodukte mit den Merkmalen des Patentanspruches 11.

Anlagen zur Vergärung von biogenen Abfällen in geschlossenen Fermentern im kontinuierlichen oder quasi-kontinuierlichen Pfropfstromverfahren für die Gewinnung von Biogas und Fermentationsprodukten werden von der Anmelderin entwickelt und seit Jahren erfolgreich betrieben und verkauft. Der Vergärungsbehälter oder Fermenter wird dabei mit dem zu vergarenden organischen Material beschickt und je nach Konsistenz und Wässergehalt unter anderem mit überschussigem Presswässer befeuchtet. Um im Fermenter optimale Bedingungen für die an der Vergärung beteiligten Mikroorganismen zu schaffen wird neben anderen Verfahrensparametern vorteilhafterweise der optimale Wässergehält überwacht und reguliert. Das Material wird durch den Fermenter gefordert und verlasst diesen in vergorenem Zustand als Nass-Gärprodukt oder Nass-Gärgut. Der Trockensubstanzanteil des Nass-Gärproduktes kann zwischen ca. 15 bis 35%, typischerweise 22 bis 27%, liegen. Da das Ausgangsmaterial der Fermentation hauptsachlich aus grob zerkleinerten biogenen Haus-, Garten- und Gewerbeabfällen besteht, können im Fermentationsprodukt noch Partikel von bis zu 15 cm Länge und/oder Durchmesser enthalten sein. So können zum Beispiel Tannen- oder Fichtenzapfen, Teile von Ästen und Wurzeln, Steinobstkerne oder auch Knochen und Steine im Digestat enthalten sein.
Die Fermenter weisen ein Volumenfassungsvermögen von bis zu 1500 m³ auf und erzielen einen Durchsatz an zu vergarendem Material von bis zu 100'000 t/Jahr. Das Nass-Gärprodukt wird am Hinterende des Fermenters aus dem Fermenter, und über Rohrleitungen zu nachgeschalteten Entwässerungsstufen gefördert. In solchen Entwässerungsstufen wird dem Nass-Gärprodukt ein Grossteil des Wässers entzogen. Erst dieses entwässerte Gärprodukt ist für die aerobe Nachrotte geeignet, die der anaeroben Vergärung im Fermenter folgen muss, um qualitativ hochwertiges Kompostmaterial zu erhalten. Damit die Nachrotte tatsächlich aerob stattfindet, darf das entwässerte Digestat weder zu feucht sein, noch darf es zu stark verdichtet sein. Der Flüssigkeitsanteil, der dem Nass-Gärprodikt in der Entwässerungsstufe entzogen wird, soll im folgenden Presswässer genannt werden. Dies unabhängig davon, ob die Flüssigkeit tatsächlich durch aktives Pressen, oder nur durch passives Abtropfen vom nassen Digestat abgetrennt wurde. Es ist offensichtlich, dass das Presswässer einen relativ hohen Trockensubstanzgehalt aufweisen kann, also oftmals dickflüssig und schlammartig ist. Wie schon oben. erwähnt, kann ein Teil des Presswässers zum Befeuchten in den Fermenter zuruckgeführt werden. Ein weiterer Teil des Presswassers kann bei Bedarf zum Befeuchten der Nachrotte eingesetzt werden.

Von der Anmelderin ist ein Verfahren bekannt, bei dem das Nass-Gärprodukt in einem ersten Schritt über Rohrleitungen zu einem Zwischenlager-Tank gepumpt wird und von dort in einem zweiten Schritt wiederum über eine Zuführleitung in eine Schneckenpresse geführt wird und dort unter relativ hohem Druck gepresst wird. Das Ueberschusswässer wird anschliessend in einem mehrstufigen Verfahren aufbereitet. In diesem Verfahren werden nach dem Pressen mittels eines Dekanters die im Ueberschusswasser vorhandenen Feststoffe von der flüssigen Phase getrennt. Flockungshilfsmittel unterstutzen dabei das Trennverfahren im Dekanter. Die im Dekanter abgeschiedenen Feststoffe bilden ein feinkorniges Material, das in einem weiteren Arbeitschritt wieder mit dem Feucht-Gärgut vermischt werden muss. Bei der Entwässerung in der Schneckenpresse wird das Gärgut stark verdichtet, was es zu einem suboptimalen Ausgangsmaterial für die Nachrotte macht.
Von der Anmelderin ist eine weitere Entwässerungsanlage bekannt, die einen Rotationsentwasserer umfasst, und uber Rohrleitungen mit dem Fermenter verbunden ist. Dem Rotationsentwässerer ist eine Mischstrecke mit Flockungsmittelbereitungsanlage vorgeschaltet. Der Austrag des Nass-Gärproduktes aus dem Fermenter und die Weiterleitung zur Mischstrecke erfolgt aktiv mittels Pumpen. Zwischen Mischstrecke und Entwässerer ist ein Zwischenspeicher angeordnet, in dem die Mischung aus Nass-Gärgut und Flockungshilfsmittel bis zur Abgabe in den Entwässerer verbleibt.
All den bekannten Entwässerungsanlagen ist gemeinsam, dass sie neben den eigentlichen Entwässerungsvorrichtungen eine Vielzahl von Rohrleitungen, Schiebern, Schleusen, Pumpen oder anderen Förderanlagen, Zwischenspeichern oder Tanks aufweisen. Dadurch wird nicht nur der Investitionsbedarf sondern auch der Platz- und Energiebedarf für die Entwässerung und Transport des Digestates erhöht. Einerseits machen die passiven Anlagenteile wie Rohrleitungen und Tanks machen den Unterhalt der Anlage aufwendiger und damit teurer, andererseits wird durch die aktiven Anteile wie Schleusen, Schieber, Pumpen, Förderer und Entwässerer der Betrieb und die Steuerung der Anlage zusätzlich verteuert und kompliziert.
Es ist die Aufgabe der Erfindung, eine Vergärungsanlage mit einer Vorrichtung zur Entwässerung und zur gleichzeitigen Forderung von Nass-Gärkompost zur Verfügung zu stellen, welche die oben genannten Nachteile nicht aufweist und mit einem Minimum an Installations- und Betriebsaufwand die Entwässerung des Nass-Gärkompostes ermöglicht. Es ist ebenfalls eine Aufgabe der vorliegenden Erfindung ein Verfahren zur Entwässerung von Nass-Gärkompost zur Verfügung zu stellen.

Diese Aufgabe lösen eine Vorrichtung mit den Merkmalen des Patentanspruches 1 und ein Verfahren mit den Merkmalen des Patentanspruches 11.

In der beiliegenden Zeichnung sind bevorzugte Ausführungsbeispiele der erfindungsgemässen Vorrichtung dargestellt, die in der nachfolgenden Beschreibung in Zusammenhang mit dem erfindungsgemässen Verfahren erläutert werden. Es zeigen
- Figur 1: einen schematischen Seitenansicht einer Vergarungsanlage mit einer Ausführungsform des Forderers/Entwässerers, wobei dessen Ummantelung teilweise weggelassen ist;
- Figur 2: eine Sicht von oben auf Teile einer Anlage gemäss Figur 1 mit kombinierter Entwässerungs- und Forderanlage;
- Figur 3: eine Seitenansicht einer Variante des Forderers/Entwässerers, wobei Spritzschutz und Siebkorb teilweise weggelassen sind; und
- Figur 4: einen Querschnitt durch eine kombinierte Entwässerungs- und Förderanlage gemäss Figur 3 entlang A-A.

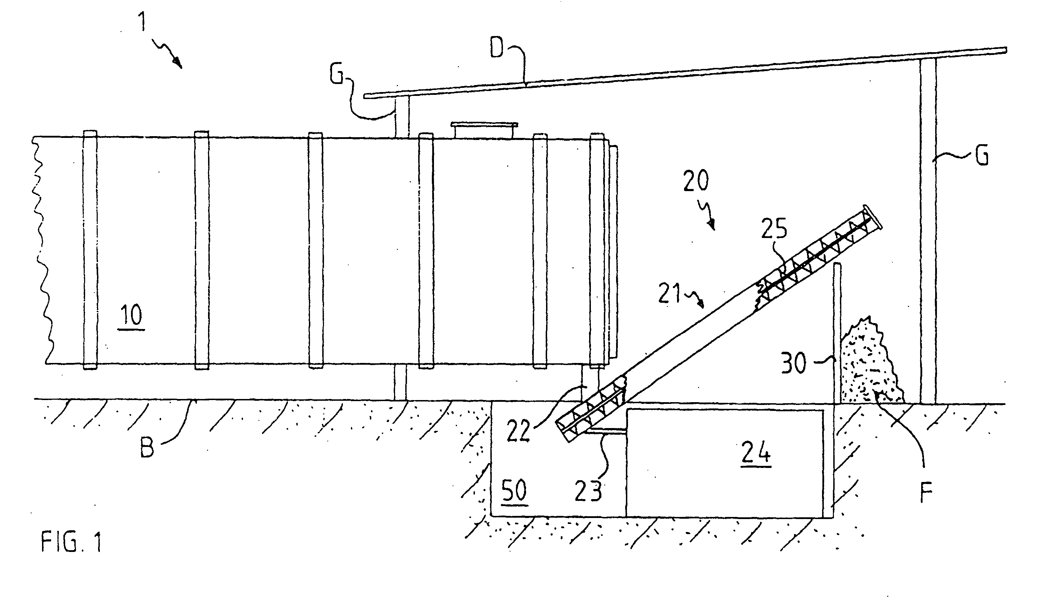

Wie in der Figur 1 skizziert, wird in einer erfindungsgemässen Entwässerungsanlage 20 einem Förderer/Entwässerer 21 aus einem Fermenter 10 stammender Nass-Gärkompost direkt zugeführt. Dies geschieht unter Ausnutzung der Schwerkraft aber auch des Vordruckes unter dem das Material aus dem im Pfropfstromverfahren betriebenen Fermenter tritt. Der natürliche Fluss des Materials wird ausgenutzt, und es sind keine Pumpen mehr notig um das Material aus dem Fermenter 10 zur Entwässerung zu transportieren. Als einziges Leitungselement ist noch ein kurzer Anschlussstutzen 22 übrig. In der schematischen Darstellung bildet dieser Anschlussstutzen 22 die Verbindung zwischen Fermenter 10 und Entwässerer 21. Dieser Anschlussstutzen kann auch vollständig weggelassen werden und der Entwässerer 21 am entsprechend ausgeformten Fermenterende angeschlossen sein. Der Nass-Gärkompost tritt am tiefsten Punkt des Fermenters 10 aus. Dadurch ist sichergestellt, dass nicht nur das vergorene biogene Material den Fermenter verlasst, sondern dass auch sedimentierte Schwerstoffe wie z.B. Kieselsteine ausgetragen werden und sich nicht im Fermenter 10 arsammeln. Der Austrag am tiefsten Punkt des liegenden Fermenters 10 bedingt, dass die Entwässerungsanlage 20 vorteilhafterweise partiell in einer Grube 50 angeordnet ist. In der in Figur 1 dargestellten Anlage ist in der Grube 50 auch ein Presswässertank 24 angeordnet. Die Tiefe Lage des Tanks 24 relativ zum Entwässerer/Förderer 21 erlaubt es das im Entwässerer/Förderer 21 anfallende Presswässer nur unter Ausnutzung der Schwerkraft über eine Presswässerleitung 23 in den Tank 24 zu leiten.
Der zylindrische Förderer/Entwässerer 21 erfullt, wie sein Name schon sagt zwei Funktionen gleichzeitig. Zum einen dient er dazu den Nass-Gärkompost soweit zu entwässern, dass die aerobe Kompostierbarkeit gewährleistet ist. Das entwässerte Digestat F muss also einen Trockensubstanzgehalt aufweisen, der hoch genug ist, dass die aerobe Nachrotte in keiner Weise negativ beeinträchtigt ist. Zum anderen transportiert der Förderer/Entwässerer 21 das Digestat vom Fermenter 10 zu einer Abgabestelle oder Abgabefläche. In der dargestellten Ausführungsform der Anlage ist ein Schneckenentwässerer 21 geneigt eingebaut. Er nimmt das Digestat etwa auf Bodenniveau B aus dem Fermenter auf und fordert es nach oben über eine Stutzwand 30. Am oberen Ende verlasst das entwässerte Gärprodukt F den Entwässerer 21 und wird auf einen Haufen F, der sich hinter der Stützmauer 30 bildet, geworfen. Hier beginnt die Nachrotte des entwässerten Digestates. Je nach Platzverhältnissen und zeitlicher Steuerung der Nachrotte, kann von der Abwurffläche aus das entwasserte Digestat auf andere Nachrotteflächen verschoben werden. Auf der Abwurffläche konnte auch ein Container stehen der das Gärgut aufnimmt.

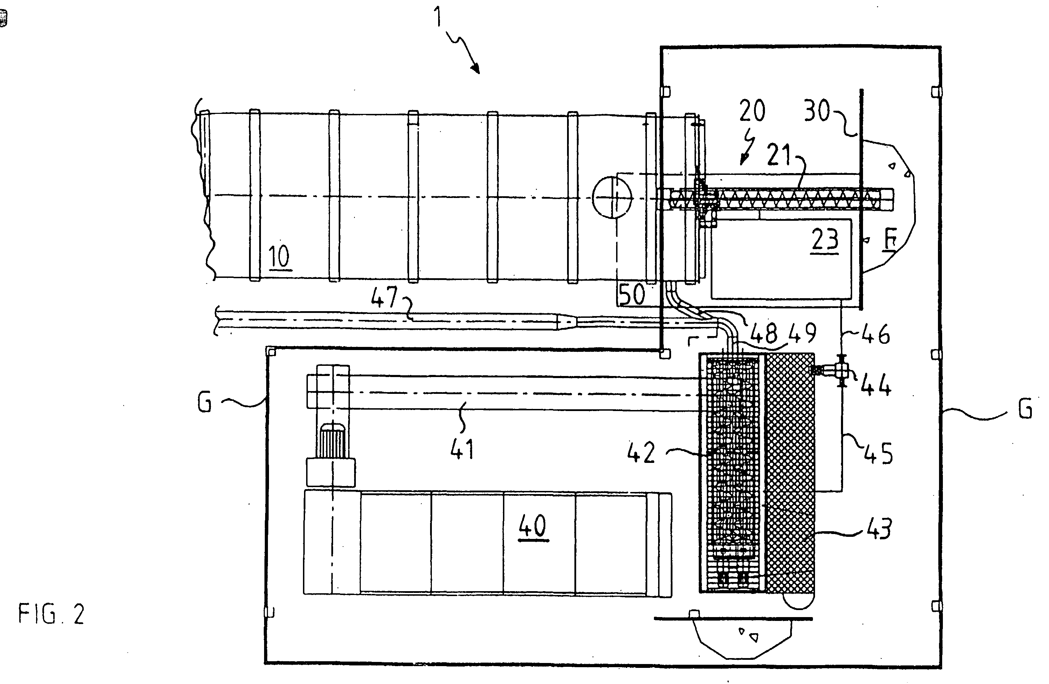

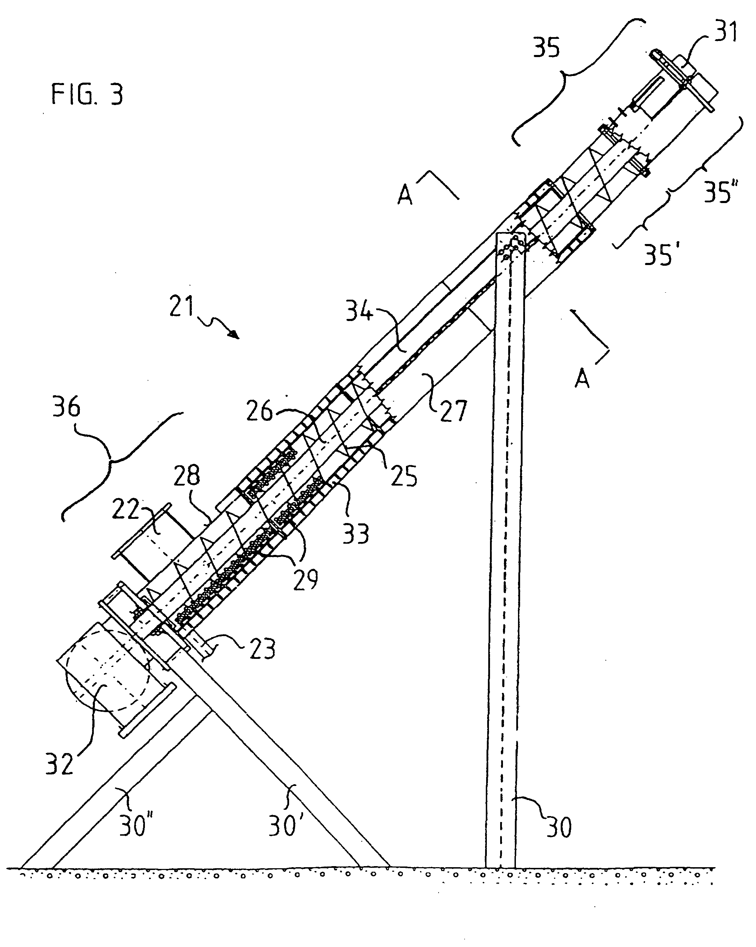

Das frische Gärprodukt und auch das Presswässer sind nicht frei von Geruchsemissionen. Um die Emissionsbelastung der Umgebung der Vergärungsanlage auf einem Minimum zu halten sind die Anlagenteile, bei denen Geruche freigesetzt werden könnten in einem geschlossenen Gebäude untergebracht. Dies ist in Figur 1 schon durch Gebäudehulle G und Dach D angedeutet, aber in Figur 2 besser ersichtlich. Hier ist gezeigt, dass der voluminöse Fermenter 10 lediglich mit seinem Ende in die Gebäudehülle G hineinragt. Die Anlagen zur Beschickung des Fermenters mit biogenem Material 40, 41, 42 und zur Animpfung des frischen Gärsubstrates mit Fermentationsprodukt 48 oder Presswässer 44, 45, 46 befinden sich ebenfalls innerhalb der Gebäudehulle. Alle Anlagenteile, respektive Verfahrensschritte, bei denen Geruchsbelastungen auftreten können sind also von der Umwelt abgeschlossen. Die Abluft aus dem Gebaude G kann uber bekannte Biofilter abgezogen und gereinigt in die Umgebung abgeblasen werden. Die Geruchsemission der Kompostieranlage wird dadurch auf ein Minimum begrenzt. Die kompakte Konstruktion der Entwässerungsanlage erlaubt es das Gebäude möglichst klein und damit möglichst kostengünstig zu halten.
Der Nass-Gärkompost weist einen Trockensubstanzanteil von ca. 15 bis 35% auf. Er wird üblicherweise kontinuierlich oder quasi-kontinuierlich aus dem Fermenter über Anschluss 22 in den Schneckenentwässerer 21 abgegeben. Die Steuerung des Entwässerers 21 ist auf diese kontinuierliche Nass-Gärgut-Zufuhr angepasst.
Der Entwässerer 21 kann in Abhängigkeit von der örtlichen Raumsituation mehr oder weniger stark geneigt sein. Der Neigungswinkel des Entwässerers sollte an die Bauhöhe des Fermenters angepasst sein.
Zwischen dem Fermenter 10 und dem Entwasserer 21 kann ein steuerbarer Sperrschieber plaziert sein, mittels dessen die Zufuhr von Nass-Gärkompost zum Entwässerer 10 bei Bedarf ganz unterbrochen werden kann.
Ein erfindungsgemässer zylindrischer Entwässerer 21, der mit Erfolg in einer Pilotanlage betrieben wird hat ein Fassungsvermögen von ca. 0.6 m³. Wie in Figur 3 dargestellt ist in einem Gehäuse 27, 28 des Entwässerers 21 auf einer Welle 26 eine Schnecke 25 rotierbar angeordnet. Die Schnecke 25 ist teilweise von einem koaxial feststehenden Siebkorb oder Sieb 29 umschlossen. Im Bereich des Einlassstutzens 22 und im Endbereich ist die Schnecke 25 von einem geschlossenen Mantel 28 umgeben. Um die Bereiche mit dem Siebkorb 29 herum ist ein Spritzschutz oder eine Auffangwanne 27 angeordnet. Das durch den Siebkorb 29 tretende Presswässer gelangt in einen Auffangraum 33 zwischen Siebkorb 29 und Spritzschutz/Auffangwanne 27. Das Presswässer kann in der Auffangwanne 27 nach unten in die Presswässerleitung 23 und zum Presswässertank 24 fliessen. Wie in Figur 3 gezeigt, ist die Schnecke 25 im vorderen Endbereich 35' des Förderers/Entwässerers 21 der Einfachheit halber nicht mit einem Lager versehen. Im vorderen Endbereich 35' ist die freifliegende Schnecke 25 wie bereits erwähnt von einem geschlossenen Mantelzylinder 28 umschlossen, an dessen Ende Staumittel 31 angebracht sind. Dieser Mantel 28 verleiht der Schnecke im Endbereich 35' genügend Führung, so dass auf eine weitere Lagerung verzichtet werden kann. Die im hinteren Endbereich 35'' angebrachten Staumittel 31 werden im weiteren noch genauer beschrieben.
Die Durchmesser des Siebes 29, des inneren Mantels 28 und der darin gelagerten Schnecke 25 sind, wie in der Figur 3 dargestellt, üblicherweise konstant über die Länge des Entwässerers 21. Das Schneckenspiel bleibt daher über die gesamte Länge des Entwässerers ebenfalls annähernd konstant. Endständig am Entwässerer sind regelbare Stauklappen 31 angeordnet. Durch die Stauklappen 31 kann der Durchmesser des Mantels massiv verringert werden. Die Schnecke 25 erstreckt sich nicht bis in den hinteren Endbereich des Mantels 35 '', das heisst bis in den Bereich der Stauklappen. Dort wird der Materialfluss des Gärgutes wird gehemmt, und es kommt zu einem Materialrückstau ausgehend vom Ende des Entwässerers 21. Der Materialpfropf, der sich im hinteren Endbereich 35'' bildet, bleibt immer nur kurzfristig bestehen und wird vom neu angeförderten Material fortwährend verdrangt. Durch die Einstellung der Stauklappen 31 lassen sich Entwässerungsgrad und Verdichtungsgrad des Gärproduktes regulieren. In einer eifachen Ausführungsform lassen sich die Stauklappen mittels einer Feder vorgespannt, passiv in einer gewünschten Stellung halten. In anderen vorteilhaften Ausführungsformen kann die Stellung der Stauklappen elektromechanisch mittels einer aktiven Steuereinheit gehalten und verandert werden. Der elektrische Antrieb der Welle 26 liefert über die Spannungsaufnahme Information über die Stärke des Materilarückstaues. Die Umdrehungsgeschwindigkeit lässt sich sehr einfach steuern.
An Stelle der Stauklappen kann der gewünschte Materialrückstau zum Beispiel mit einem auf der Welle stehend gelagerten Konus erzeugt werden. Andere Mittel zum regulieren des Mantelquerschnittes sind bekannt und sollen hier nicht weiter beschrieben werden.
Im Eintragsbereich 36 des Entwässerers 21 werden die Schneckengange achon alleine durch den Vordruck des Fermenters und die Schwerkraft mit dem nassen Gärprodukt gefüllt. Durch die Drehbewegung der Schnecke 25 wird das Material entlang der inneren Mantelwand 28 respektive entlang des Siebkorbes 29 geführt. Die Entwässerung erfolgt dabei teilweise passiv, das heisst, das Presswässer kann abtropfen während es weitergefordert wird. Durch den Wässerverlust nimmt auch das Materialvolumen im Schneckengang ab. Dies erhöht sich jedoch wieder zum Austragsbereich des Entwässerers 21 hin durch den von den Stauklappen bewirkten Materialrückstau.

Die Siebflächen 29 sind vorzugsweise aus Edelstahl gefertigt, so dass sie vom alkalischen Gärkompost nicht angegriffen werden und eine optimale Lebensdauer bei geringem Wartungsaufwand gewährleistet ist. Da das zu entwässernde Material kontinuierlich entlang der Siebflächen 29 gefördert wird, kann sich kein Filterkuchen aufbauen und es kommt während des Betriebes zu keinem ungewünschten lokalen Anstieg des Filtrationswiderstandes. In bevorzugten Ausführungsformen der Erfindung können die Schneckengeometrie, die Form und die Lochweite des Siebes 29 so aufeinander abgestimmt werden, dass es in Abhängigkeit von der Stellung der Stauklappen 31 zu einem kontinuierlichen sanften Druckanstieg in Forderrichtung kommt. Dadurch wird über die gesamte Länge eine gute Entwässerungsleistung gewährleistet. Der Aufbau von hohen Drücken wird gleichzeitig vermieden.
Allgemein gilt, dass ein Teil der Entwässerungsleistung im Anfangsbereich des Entwässerers 21 schon allein durch das passive schwerkraftgetriebene Abtropfen des Gärproduktes erbracht wird. Zusätzlich kann der Vordruck des aus dem Fermenter 10 austretenden Materials zur initialen Entwässerung genutzt werden. Dadurch lässt sich das Antriebsaggregat 32 für den Entwässerer klein und der Energiebedarf für seinen Betrieb niedrig halten. Das Digestat wird durch die kontinuierliche Umwälzung bei der Forderung im Entwässerer nicht in unerwünschter Weise verdichtet, was sich positiv auf die Nachrotte und damit auf die Kompostqualität auswirkt.

Um den Wartungsaufwand niedrig zu halten und einen möglichst kontinuierlichen Betrieb zu gewahrleisten, kann der Entwässerer 21 mit einer automatischen Rückspüleinrichtung für das Sieb 29 ausgestattet sein.

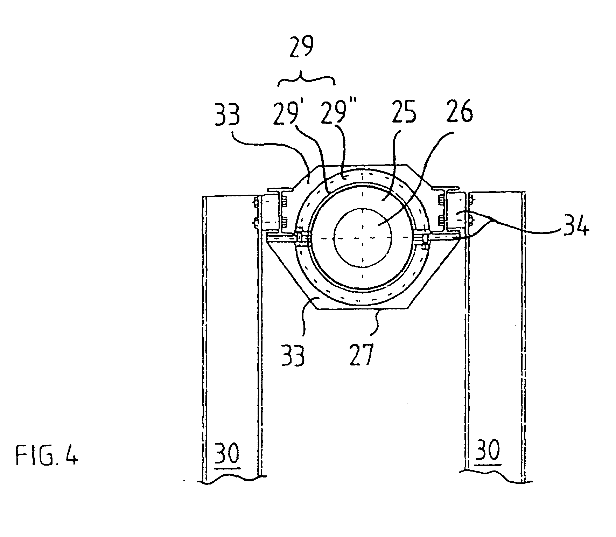

Die Figur 4 zeigt den Förderer/Entwässerer gemäss Figur 3 im Schnitt A-A. Zur Stabilisierung sind seitlich entlang des Entwässerers Holme 34 angebracht, an denen der Siebkorb 29 befestigt ist. Der Siebkorb 29 umfasst ein Siebblech 29' das um die Schnecke 25 angeordnet ist und einen Tragkorb 29'' der dem Siebblech 29' die notige mechanische Stabilität verleiht. Der Siebkorb 29 besteht aus mehreren Halbschalen, die entsprechend der gewünschten Länge aneinandergereiht sind. Das durch den Siebkorb 29 tretende Presswässer wird vom Spritzschutz 27, der ebenfalls an den seitlichen Holmen 34 befestigt ist, aufgefangen und abgeleitet. Der kombinierte Förderer/Entwässerer 21 kann wie in den Figuren 3 und 4 durch zusätzliche Stutzen 30, 30' und 30'' stabilisiert und in Position gehalten werden. In einer hier nicht dargestellten vorteilhaften Ausführungsform ist der Förderer/Entwässerer 21 schwenkbeweglich am Fermenter, angebracht. Durch geeignete Anpassungen zum Beispiel des Anschlussstutzens 22 kann eine Schwenkbeweglichkeit um eine Vertikalachse im Bereich des Stutzens 22 erreicht werden. Gerade bei kleineren hier nicht dargestellten Anlagen konnen so durch blosses Schwenken des Förderers/Entwässerers 21 zum Beispiel verschiedene Nachrotte-Kompartimente nacheinander mit Digestat gefüllt werden.

Anstelle der in Figuren 3 und 4 gezeigten Stützen 30, 30', 30'' kann in einer solchen Ausführungsform der Förderer/Entwässerer 21 über einen Seilzug an einem Kran oder am oberen Bereich des Fermenterendes befestigt sein. Analog zur Verschwenkung in der horizontalen kann natürlich auch eine Auf und Abbeweglichkeit der Austrittsöffnung des Förderers/Entwässerers 21 in der Vertikalen erreicht werden.
In einer Anlage gemäss der vorliegenden Erfindung kann also auf den Grossteil der, in den bisher bekannten Anlagen zur Entwässerung und zum Materialtransport nötigen, Elemente verzichtet werden. Das fertige Digestat muss nicht mehr aktiv aus dem Fermenter 10 abgesaugt oder abgepumpt werden, sondern es wird der im Fermenter 10 herrschende Vordruck genutzt um das Material auszutragen. Dieser Vordruck wird dann unmittelbar anschliessend für die erste Entwässerung im Förderer/Entwässerer 21 genutzt. Dies ist erst dadurch möglich, dass der Förderer/Entwässerer 21 unmittelbar am Fermenter angeschlossen ist und dadurch die Druck- und Reibungsverluste minimiert sind. Alle bisher üblichen Leitungen und Fördereinrichtungen, die für den Transport des nassen Materials zur und innerhalb der Entwässerungsanlage und anschliessend zur Nachrotte nötig waren, entfallen. Das Digestat wird nicht mehr wie bisher mit hohem Aufwand vom Fermenter zu einer davon entfernt installierten Entwässerungsanlage, mit womoglich mehreren Entwässerungsstufen, und anschliessend weiter zur Nachrotte transportiert, sondern Transport und Entwässerung sind räumlich und zeitlich zu einer Einheit zusammengefasst.

## Patentansprüche

1. Anlage (1) zum Vergaren von biogenen Abfallstoffen welche mindestens einen Fermenter (10), mindestens eine Entwässerungsanlage und mindestens eine Fördereinrichtung zum Fördern des aus dem Fermenter stammenden Nass-Gärgutes umfasst, **dadurch gekennzeichnet, dass** die Entwässerungsanlage und die Fördereinrichtung zu einem kombinierten Förderer/Entwässerer (21) zusammengefasst sind, der direkt an eine Austrittsöffnung am Hinterende des Fermenters (10) angeschlossen ist, und dass das vergorene Nass-Gärgut mittels des Förderers/Entwässerers (21) gefördert und gleichzeitig entwässert wird.

2. Anlage (1) gemäss Anspruch 1, **dadurch gekennzeichnet, dass** sich der Förderer/Entwässerer (21) entlang einer linearen Achse erstreckt.

3. Anlage (1) gemass Anspruch 1, **dadurch gekennzeichnet, dass** der Förderer/Entwässerer (21) ein Schnecken-Förderer/Entwässerer (21) ist.

4. Anlage (1) gemäss Anspruch 3, **dadurch gekennzeichnet, dass** in einem Gehause (27, 28) des Förderers/Entwässerers (21) auf einer Welle (26) eine Schnecke (25) rotierbar angeordnet ist und die Schnecke (25) zumindest teilweise von einem koaxial feststehenden Siebkorb oder Sieb (29) umschlossen ist.

5. Anlage nach Anspruch 4, **dadurch gekennzeichnet, dass** der Förderer/Entwässerer (21) eine automatische Spuleinrichtung zum Reinigen und/oder Rückspülen des Siebes (29) aufweist.

6. Anlage (1) gemass einem der Ansprüche 1 bis 5, **dadurch gekennzeichnet, dass** an einem Auslassende des Förderers/Entwässerers (21) Mittel zum Rückstauen (31) des geförderten Digestates angebracht sind.

7. Anlage (1) gemäss Anspruch 6, **dadurch gekennzeichnet, dass** die Mittel zum Rückstauen mindestens eine passiv oder aktiv einstellbare oder steuerbare Stauklappe (31) umfassen, wobei mittels der mindestens einen Stauklappe (31) der Mantelquerschnitt und damit der Materialfluss veranderbar ist.

8. Anlage nach einem der Anspruche 1 bis 7, **dadurch gekennzeichnet, dass** der Förderer/Entwässerer (21) schwenkbeweglich am Fermenter (10) angeordnet ist.

9. Anlage (1) gemass Anspruch 1, **dadurch gekennzeichnet, dass** sich die Austrittsoffnung am tiefsten Punkt des Fermenters (10) befindet und dass ein Einlassbereich (36) des Förderers/Entwässerers (21) unter dem Fermenter (10) angeordnet ist.

10. Anlage nach Anspruch 1, **dadurch gekennzeichnet, dass** der Förderer/Entwässerer (21) einen Anschlussstutzen (22) zum Anschluss an den Fermenter (10) aufweist.

11. Verfahren zum Fordern und Entwässern von aus einem im Pfropfstrombetrieb arbeitenden Fermenter (10) stammenden Gärprodukten bei dem das nasse Gärprodukt bis zur aeroben Kompostierfähigkeit entwässert wird und das zu entwässernde Gärprodukt aus einem Fermenter durch mindestens eine Transportanlage einer Entwässerungsanlage und der Nachrotte zugeführt wird, **dadurch gekennzeichnet, dass** Entwässerung und Materialtransport in einem einzigen kombinierten Verfahrensschritt durchgeführt werden.

12. Verfahren gemass Anspruch 11 **dadurch gekennzeichnet, dass** der Vordruck des aus dem Fermenter (10) austretenden Nass-Gärgutes in einer ersten aktiven Entwässerungsphase zur Entwässerung in einem Eintragsbereich des Förderers/Entwässerers (21) genutzt wird.

13. Verfahren gemäss Anspruch 12 **dadurch gekennzeichnet, dass** während der ersten aktiven Entwässerungsphase der Eintragsbereich des Förderers/Entwässerers (21) annähernd vollständig mit Digestat gefüllt wird.

14. Verfahren gemäss Anspruch 11 **dadurch gekennzeichnet, dass** Förderung und Entwässerung entlang einer linearen Achse (26) ablaufen.

## Claims

1. An installation (1) for fermenting biogenic waste substances which comprises at least one fermenter (10), at least one drainage installation and at least one conveyor means for conveying the wet fermentation product coming from the fermenter, **characterised in that** the drainage installation and the conveyor means are grouped together into a combined conveyor/drainer (21) which is connected directly to an exit opening at the rear end of the fermenter (10), and that the fermented wet fermentation product is conveyed and simultaneously drained by way of the conveyer/drainer (21).

2. An installation (1) according to claim 1, **characterised in that** the conveyor/drainer (21) extends along a linear axis.

3. An installation according to claim 1, **characterised in that** the conveyor/drainer (21) is a worm conveyor/drainer (21).

4. An installation (1) according to claim 3, **characterised in that** in a housing (27, 28) of the conveyor/drainer (21) a worm (25) is rotatably arranged on a shaft (26), and the worm (25) at least partly is enclosed by a coaxially stationary sieve basket or sieve (29).

5. An installation according to claim 4, **characterised in that** the conveyor/drainer (21) has an automatic rinsing means for cleaning and/or back-rinsing the sieve (29).

6. An installation (1) according to one of the claims 1 to 5, **characterised in that** at one outlet end of the conveyor/drainer (21) there are attached means for damming (31) the conveyed digestate.

7. An installation (1) according to claim 6, **characterised in that** the means for damming comprise a passively or actively adjustable or controllable damming flap (31), wherein by way of the at least one damming flap (31) the casing cross section and thus the material flow may be varied.

8. An installation according to one of the claims 1 to 7, **characterised in that** the conveyor/drainer (21) is arranged pivotally movable on the fermenter (10).

9. An installation (1) according to claim 1, **characterised in that** the exit opening is located at the lowest point of the fermenter (10) and that an inlet region (36) of the conveyor/drainer (21) is arranged below the fermenter (10).

10. An installation according to claim 1, **characterised in that** the conveyor/drainer (21) comprises a connection union (22) for connection to the fermenter (10).

11. A method for conveying and draining fermentation products coming from a fermenter (10) functioning with the plug-flow operation, with which the wet fermentation product is drained until aerobic compostability and the fermentation product to be drained is supplied from a fermenter by way of at least one transport installation to a draining installation and to the secondary compost, **characterised in that** the draining and the material transport is carried out in a single combined method step.

12. A method according to claim 11, **characterised in that** the pre-pressure of the wet fermentation product exiting the fermenter (10) is used in a first active draining phase for draining in a feed region of the conveyor/drainer (21).

13. A method according to claim 12, **characterised in that** during the first active drainage phase, the feed region of the conveyor/drainer (21) is approximately completely filled with digestate.

14. A method according to claim 11, **characterised in that** the conveying and drainage occur along a linear axis (26).

## Revendications

1. Installation (1) pour la fermentation de déchets biogènes laquelle comprend au moins un fermenteur (10), au moins une installation de déshydratation et au moins un dispositif de transport visant le transport du produit de fermentation trempé issus du fermenteur; cette installation étant **caractérisée par le fait que** l'installation de déshydratation et le dispositif de transport sont groupés à un transporteur / déshydratant (21) combiné qui est directement attaché au fond arrière du fermenteur (10) et que le produit de fermentation, trempé et fermenté, est transporté au moyen du transporteur / déshydratant (21) et est en même temps déshydraté.

2. Installation selon la revendication 1, **caractérisée par le fait que** le transporteur / déshydratant (21) s'avance le long d'un axe linéaire.

3. Installation selon la revendication 1, **caractérisée par le fait que** transporteur / déshydratant (21) est un transporteur / déshydratant (21) hélicoïdal.

4. Installation selon la revendication 3, **caractérisée par le fait qu'**une hélice (25) est disposée de façon à pouvoir tourner sur un axe (25) dans un récipient (27, 28) du transporteur / déshydratant (21) et l'hélice (25) est entourée, au moins en partie, par un panier d'essorage en fil perforé ou par un filtre (29) coaxial fixe.

5. Installation selon la revendication 4, **caractérisée par le fait que** transporteur / déshydratant (21) présente un dispositif de rinçage pour le nettoyage et/ou le lavage à contre courant du filtre (29).

6. Installation selon une des revendications de 1 à 5, **caractérisée par le fait que** des moyens pour la retenue (31) du digestat transporté sont ajoutés à une décharge du transporteur / déshydratant (21).

7. Installation selon la revendication 6, **caractérisée par le fait que** les moyens pour la retenue comprennent au moins une vanne de retenue (31) passive ou active réglable ou contrôlable, auquel cas, au moyen d'au moins une vanne de retenue (31), la section transversale de la surface et avec cela l'écoulement du matériel sont variables.

8. Installation selon une des revendications de 1 à 7 **caractérisée par** le fait le transporteur / déshydratant (21) est disposé au fermenteur de façon à pouvoir se mouvoir par mouvements pivotants.

9. Installation selon la revendication 1, **caractérisée par le fait que** l'orifice de décharge se trouve au point le plus bas du fermenteur (10) et qu'un champ d'enchâssement (36) du transporteur / déshydratant (21) est disposé sous le fermenteur (10).

10. Installation selon la revendication 1, **caractérisée par le fait que** le transporteur / déshydratant (21) montre un tuyau de raccordement (22) pour le raccordement au fermenteur (10).

11. Procédé pour transporter et déshydrater des produits de fermentation issus d'un fermenteur (10) fonctionnant en écoulement à noyau, et lors de ce procédé, le produit de fermentation trempé est déshydraté jusqu'à la capacité de compostage anaérobie et le produit de fermentation à déshydrater est transporté d'un fermenteur, par le biais d'au moins une installation de transport, à une installation de déshydratation et à un dispositif de post-compostage; ce procédé étant **caractérisé par le fait que** la déshydratation et le transport de matériel sont exécutés dans une seule étape du procédé.

12. Procédé selon la revendication 11, **caractérisé par le fait que** la pression d'admission des produits de fermentation sortant du fermenteur (10) est d'utilité dans une première phase active de déshydratation pour la déshydratation dans un secteur d'alimentation du transporteur / déshydratant (21).

13. Procédé selon la revendication 12, **caractérisé par le fait que** pendant la première phase active de déshydratation, le secteur d'alimentation du transporteur / déshydratant (21) est rempli à peu près entièrement avec du digestat.

14. Procédé selon la revendication 11, **caractérisé par le fait que** le transport et la déshydratation s'écoulent le long d'un axe (26) linéaire.
